# EUROPÄISCHE PATENTSCHRIFT

(11) **EP 1 455 655 B1**
(45) Veröffentlichungstag und Bekanntmachung des Hinweises auf die Patenterteilung: **14.06.2006**
(21) Anmeldenummer: 02783081.9
(22) Anmeldetag: 11.11.2002
(51) Int. Cl.: A61B 17/04

(54) **KNOCHENANKER**
BONE ANCHOR
SYSTEME D'ANCRAGE OSSEUX

(30) Priorität: 17.12.2001 DE 10161970
(43) Veröffentlichungstag der Anmeldung: 15.09.2004
(73) Patentinhaber: Tutogen Medical GmbH, 91077 Neunkirchen am Brand (DE)
(72) Erfinder: GOTZEN, Leo, 35041 Marburg (DE)
(74) Vertreter: Manitz, Finsterwald & Partner GbR
(86) Internationale Anmeldenummer: PCT/EP2002/012588
(87) Internationale Veröffentlichungsnummer: WO 2003/051205

(56) Entgegenhaltungen:
- WO-A-01/06933
- DE-U- 20 002 901
- US-A- 5 879 372
- US-A1- 2001 041 916

## Beschreibung

Die vorliegende Erfindung betrifft einen Knochenanker zur Refixierung von Weichgewebe an Knochen mit Hilfe eines Fadens. Ein solcher Anker ist z.B. aus WO-A-0106933 bekannt.

Derartige Knochenanker sind bisher nur aus Metall oder Kunststoffen bekannt und dienen dazu, nach einer Ruptur von Weichgeweben (Bändern, Sehnen und Muskeln) vor allem im Bereich der Gelenke, eine Refixation des Weichgewebes an Knochen zu erreichen. Hierzu werden die Anker in einer Bohrung des Knochens versenkt, wobei an dem Anker ein Faden fixiert ist, mit dem das abgerissene Weichgewebe wieder am Knochen fixiert werden kann.

Es ist die Aufgabe der vorliegenden Erfindung, einen Knochenanker zu schaffen, der besonders verträglich ist und der in einem Knochen besonders fest verankerbar ist.

Die Lösung dieser Aufgabe erfolgt durch die Merkmale des Anspruchs 1 und insbesondere dadurch, dass der Knochenanker aus einem einstückigen, im Wesentlichen zylindrischen Körper aus menschlichem oder tierischem kortikalem Knochen besteht, der mindestens zwei Durchgangsbohrungen mit jeweils zwei Öffnungen aufweist, wobei eine Öffnung einer Bohrung benachbart zu einer Öffnung der anderen Bohrung angeordnet ist.

Der erfindungsgemäße Knochenanker zeichnet sich einerseits durch ausgezeichnete Biokompatibilität und Osteointegration aus, da der Knochenanker aus natürlichem Knochenmaterial hergestellt ist, das besonders gut mit dem umgebenden Knochenmaterial des Patienten verwachsen kann, ohne dass hierdurch Störungen auftreten. Gleichzeitig ist der Knochenanker aufgrund des verwendeten kortikalen bzw. kompakten Knochens sehr stabil, das heißt der erfindungsgemäße Knochenanker weist eine ebenso große Ausreißsicherheit auf, wie vergleichbare Metallanker.

Schließlich lassen sich die erfindungsgemäßen Knochenanker auf besonders einfache Weise sehr fest und sicher in der Bohrung eines umgebenden Knochens fixieren. Zu diesem Zweck wird der Faden zur Refixierung des Weichgewebes so durch die beiden Bohrungen des Knochenankers geführt, dass zwei freie Fadenenden jeweils aus den beiden benachbarten Öffnungen der zwei Bohrungen austreten. Auf diese Weise ist es möglich, nach Einbringen des Knochenankers in die Bohrung des Patientenknochens an den beiden freien Fadenenden ruckartig zu ziehen, woraufhin auf den Knochenanker ein Drehmoment ausgeübt wird. Ist dieses groß genug, so wird der Knochenanker in dem umgebenden spongiösen Knochen des Patienten leicht gedreht und dadurch verkantet. Auf diese Weise ist der Anker sicher verankert, so dass sich dieser nicht mehr aus der Bohrung löst.

Vorteilhafte Ausführungsformen der Erfindung sind in den Unteransprüchen, der Beschreibung und den Zeichnungen beschrieben.

Nach einer ersten vorteilhaften Ausführungsform können die beiden benachbarten Öffnungen im Bereich einer Kante des Körpers, z.B. einer Vorderkante, angeordnet sein. Wird der Knochenanker mit dieser Kante voran in die zugehörige Bohrung eingesetzt, so ist das Dreh- bzw. Kippmoment besonders groß, das entsteht, wenn an den in oben beschriebener Weise eingefädelten Fadenenden ruckartig gezogen wird.

Nach einer weiteren vorteilhaften Ausführungsform kann sich der Körper an seinem vorderen Ende verjüngen, wobei die beiden benachbarten Öffnungen im Bereich des vorderen Endes angeordnet sein können. Bei dieser Ausführungsform ist ein besonders einfaches und sicheres Einbringen des Knochenankers in die Bohrung aufgrund des sich verjüngenden vorderen Endes gewährleistet. Gleichzeitig ist das entstehende Kipp- bzw. Drehmoment maximiert.

Es ist vorteilhaft, wenn eine Bohrung quer zur Längsachse des Körpers verläuft, da auf diese Weise die Schwächung des Grundkörpers durch die Bohrung minimiert ist. Ebenso ist es vorteilhaft, wenn beide Bohrungen unter einem Winkel zueinander verlaufen, da dies ebenfalls die Schwächung des Körpers minimiert, wodurch die Festigkeit des Körpers erhöht ist. Ein besonders stabiler Knochenanker ergibt sich, wenn beide Bohrungen unter einem Winkel von etwa 45° zueinander verlaufen, da in diesem Fall-ein Kompromiss zwischen Lage und Abstand der beiden Bohrungen geschaffen ist.

Weiter ist es sowohl für den Fadenverlauf wie auch für die Stabilität des Knochenankers vorteilhaft, wenn die beiden Bohrungen im Wesentlichen in einer Ebene liegen.

Am Außenumfang des Körpers kann zwischen zwei Öffnungen zweier Bohrungen eine Rille vorgesehen sein. Durch eine solche Außenrille ist gewährleistet, dass der in den Knochenanker eingefädelte Faden bei Einbringen des Knochenankers in die zugehörige Bohrung, beispielsweise mittels eines Presssitzes, nicht beschädigt wird oder abreißt. Hierbei ist es besonders vorteilhaft, wenn sich diejenige Rille, in welche die beiden benachbarten Öffnungen münden, bis zur oberen Stirnseite des Körpers erstreckt, da hierdurch ein Austrittskanal für die beiden Fadenenden geschaffen ist. Genauso ist es vorteilhaft, wenn sich diejenige Rille, die die beiden anderen Öffnungen der Bohrungen verbindet, nur zwischen diesen beiden Öffnungen erstreckt, da hierdurch der Körper des Knochenankers am wenigsten geschwächt wird. Aus dem gleichen Grund kann die Breite dieser Rille minimiert werden, wohingegen die Breite der sich bis zum hinteren Ende des Körpers erstreckenden Rille in vorteilhafter Weise genauso breit wie die Breite der Öffnungen gewählt wird, da hierdurch sichergestellt ist, dass der Faden bei Einbringen des Knochenankers in die Bohrung nicht zwischen Außenwand des Knochenankers und Bohrung verklemmt und dadurch unter Umständen abreißt.

Bevorzugt sind am Außenumfang des Körpers umlaufende Rillen vorgesehen, die konzentrisch zur Längsachse des Körpers verlaufen. Auf diese Weise kann der Knochenanker mittels Presssitz in eine Knochenbohrung eingebracht werden, die einen geringfügig geringeren Durchmesser als der Außendurchmesser des Ankers besitzt.

Nachfolgend wird die vorliegende Erfindung rein beispielhaft anhand vorteilhafter Ausführungsformen und unter Bezugnahme auf die beigefügten Zeichnungen beschrieben. Es zeigen:
- Fig. 1: einen Längsschnitt durch einen Knochenanker sowie einen Querschnitt durch den Knochenanker entlang der gestrichelten Linie;
- Fig. 2: eine Seitenansicht des Knochenankers von Fig. 1;
- Fig. 3: eine weitere Seitenansicht des Knochenankers von Fig. 1 und 2;
- Fig. 4: eine weitere Ausführungsform eines Knochenankers, der mit einem Faden versehen und in eine Knochenbohrung eingebracht ist; und
- Fig. 5: den Knochenanker von Fig. 4 in vollständig verankertem Zustand.

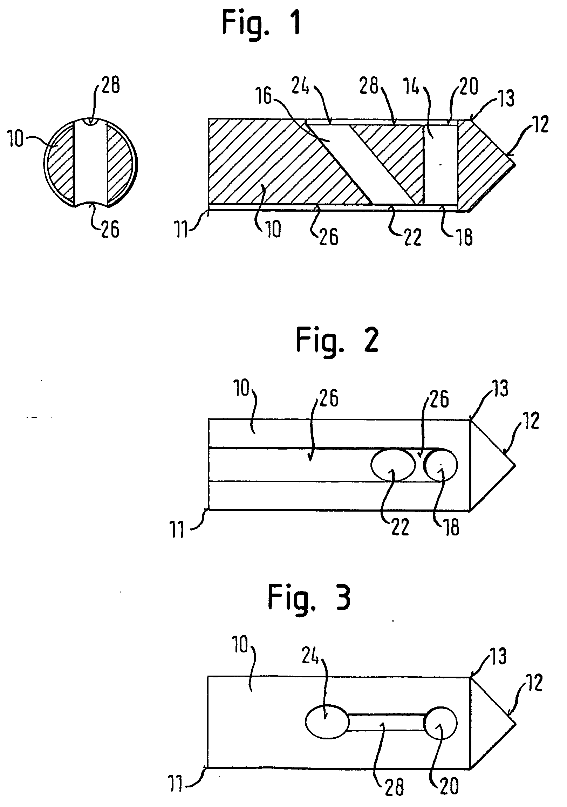

Der in den Fig. 1 bis 3 dargestellte Knochenanker besteht aus einem einstückigen, im Wesentlichen zylindrischen Körper 10 aus konserviertem menschlichem oder tierischem kortikalem Knochen. Der Körper ist länglich und kreiszylindrisch ausgebildet, wobei das vordere Ende 12 des Körpers sich konisch verjüngt. Der Spitzenwinkel zur Längsachse des Körpers beträgt hier etwa 45°. Der Übergang zwischen dem kreiszylindrischen Abschnitt und der konischen Spitze des Körpers definiert eine Kante 13.

Der Körper 10 weist zwei Durchgangsbohrungen 14 und 16 auf, wobei die Durchgangsbohrung 14 zwei Öffnungen 18 und 20 und die Bohrung 16 zwei Öffnungen 22 und 24 aufweist. Beide Bohrungen 14 und 16 besitzen den gleichen Durchmesser. Die Bohrung 14 verläuft rechtwinklig zur Längsachse des Körpers 10. Die Bohrung 16 verläuft unter einem Winkel von etwa 45° zur Längsachse der Bohrung 14, wobei beide Bohrungen 14 und 16 in der gleichen Ebene liegen.

Wie insbesondere Fig. 2 zeigt, ist die Öffnung 18 der Bohrung 14 am vorderen Ende des Körpers 10 benachbart zu der Öffnung 22 der Bohrung 16. Beide Öffnungen 18 und 22 sind um nur wenige Millimeter voneinander beabstandet und liegen nahe bei der vorderen Kante 13.

Fig. 3 zeigt, dass aufgrund der schrägen Orientierungen der beiden Bohrungen 14 und 16 zueinander, die beiden anderen Öffnungen 20 und 24 deutlich voneinander beabstandet sind, das heißt die Öffnung 20 liegt ebenfalls im Bereich des vorderen Endes des Körpers 10, wohingegen die Bohrung 24 etwa im Bereich des oberen Drittels des Körpers 10 liegt.

Die Fig. 1 bis 3 zeigen ferner, dass jeweils zwei Öffnungen 18 und 22 sowie 20 und 24 durch eine am Außenumfang des Körpers 10 vorgesehene Rille mit konkavem Querschnitt verbunden sind. Fig. 2 zeigt, dass die beiden benachbarten Öffnungen 18 und 22 durch eine Rille 26 miteinander verbunden sind, die sich bis an das hintere Ende bzw. die hintere Kante 11 des Körpers 10 erstreckt. Demgegenüber zeigt Fig. 3, dass die beiden Öffnungen 20 und 24 durch eine Rille 28 miteinander verbunden sind, die sich nur zwischen den beiden Öffnungen 20 und 24 erstreckt. Beide Rillen 26 und 28 verlaufen am Außenumfang des Körpers 10 parallel zu dessen Längsachse, wobei die Breite der Rille 26 dem Durchmesser der Bohrungen 14, 16 entspricht und die Breite der Rille 26 etwa 50 % des Durchmessers der Bohrungen 14, 16 beträgt.

Der in den Fig. 1 bis 3 dargestellten Knochenanker kann eine Länge von beispielsweise etwa 8 bis 16 mm und einen Durchmesser von beispielsweise etwa 2 bis 5 mm aufweisen. Der Durchmesser der Bohrungen kann beispielsweise zwischen etwa 0,8 und 1,5 mm variieren. Die Breite der Rillen kann zwischen etwa 0,5 und 1,6 mm variieren. Selbstverständlich sind hier auch andere Grunddimensionen möglich. Vorteilhaft ist in jedem Fall, wenn der Körper des Knochenankers im Wesentlichen länglich und hohlzylindrisch ausgebildet ist.

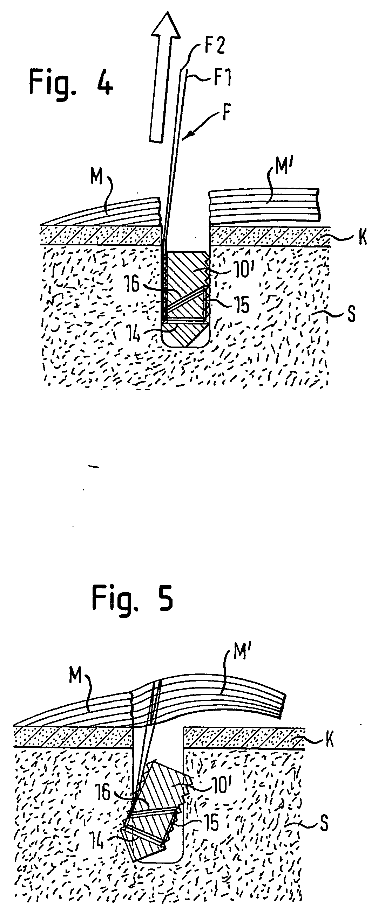

Die Fig. 4 und 5 zeigen eine weitere Ausführungsform eines Knochenankers 10', die sich jedoch von der in den Fig. 1 bis 3 dargestellten Ausführungsform lediglich dadurch unterscheidet, dass am Außenumfang des Körpers 10' eine Vielzahl von umlaufenden und zur Längsachse konzentrischen Rillen 15 vorgesehen ist, die ein Fixieren des Knochenankers in der zugehörigen Bohrung mittels Presssitz begünstigt.

Wie die Fig. 4 und 5 zeigen, ist ein Faden F durch die Bohrungen 14 und 16 so hindurchgeführt, dass die beiden freien Fadenenden F1 und F2 jeweils durch die beiden benachbarten Öffnungen 18 und 22 austreten. Hierzu wird der Faden F mit einem Fadenende in die Öffnung 18, dann durch die Bohrung 14 hindurch aus der Öffnung 20 heraus, anschließend durch die Öffnung 24 in die Bohrung 16 hinein und zum Schluss durch die Öffnung 22 aus der Bohrung 16 herausgeführt, so dass die beiden freien Fadenenden F1 und F2 in der Rille 26 zum hinteren Ende des Knochenankers im Bereich der Kante 11 des Körpers 10' geführt werden können.

Die Fig. 4 und 5 zeigen den Querschnitt durch einen menschlichen Knochen mit Kortikalis K und Spongiosa S. Oberhalb der Kortikalis befindet sich ein Muskelansatz M, von dem ein Stück M' abgerissen ist.

Zum Fixieren des Knochenankers 10' wird in den Knochen eine Bohrung eingebracht, die so tief ist, dass der Knochenanker 10' unterhalb der Kortikalis liegt, wenn dieser vollständig im Bohrloch versenkt ist. Das Bohrloch wird geringfügig kleiner als der Querschnitt des Knochenankers 10' gewählt, so dass der Knochenanker 10' wie in Fig. 4 gezeigt, mittels Presssitz in dem Bohrloch verankert werden kann. Hierbei ist der Faden F mit seinen beiden freien Fadenenden F 1 und F2 in der Rinne 26 geführt, so dass dieser nicht zwischen Knochenanker 10' und dem umgebenden Bohrloch gequetscht werden kann.

Nachdem der Knochenanker wie in Fig. 4 gezeigt, in die Bohrung des Knochens eingebracht ist, wird an beiden Fadenenden F1 und F2 zugleich mit einem starken Ruck in Richtung des in Fig. 4 eingezeichneten Pfeiles gezogen. Hierdurch wird auf dem Knochenanker 10' ein Drehmoment bzw. ein Kippmoment ausgeübt, so dass dieser wie in Fig. 5 gezeigt, in der Spongiosa S gedreht und darin verkantet wird. Hierdurch ist der Knochenanker 10' sicher verankert und gegen ein Herausreißen gesichert. Anschließend kann mit Hilfe des Fadens F das abgerissene Muskelstück M' festgenäht werden, so dass dieses wieder mit dem Knochen verwachsen kann.

Die erfindungsgemäßen Knochenanker können durch Autoklavieren oder durch Gammastrahlen sterilisiert werden. Eine Sterilisation durch Anwendung von feuchter Hitze in der Größenordnung von 120°C für eine Dauer von 20 Minuten und einem anschließenden Trocknen auf 60 bis 50°C innerhalb von 30 Minuten hat sich als vorteilhaft herausgestellt.

### Bezugszeichenliste

- 10, 10': Körper
- 11: Kante
- 12: vorderes Ende
- 13: Kante
- 14: Bohrung
- 15: Rillen
- 16: Bohrung
- 18; 20: Öffnung
- 22,24: Öffnung
- 26, 28: Rille

- F: Faden
- F1, F2: Fadenenden
- K,: Kortikalis
- M: Muskelgewebe
- M': abgerissenes Stück
- S: Spongiosa

## Patentansprüche

1. Knochenanker zur Refixierung von Weichgewebe (M') an Knochen (K, S) mit Hilfe eines Fadens (F), bestehend aus einem einstückigen, länglichen und im Wesentlichen zylindrischen Körper (10) aus menschlichem oder tierischem kortikalem Knochen, dessen vorderes Ende (12) sich konisch verjüngt, und der mindestens zwei Durchgangsbohrungen (14, 16) mit jeweils zwei Öffnungen (18, 20; 22, 24) aufweist, wobei
- beide Bohrungen (14, 16) in einer Ebene liegen und unter einem Winkel zueinander verlaufen,
- eine Öffnung (18) einer Bohrung (14) benachbart zu einer Öffnung (22) der anderen Bohrung (16) angeordnet ist,
- die andere Öffnung (20) der einen Bohrung (14) beabstandet zu der anderen Öffnung (24) der anderen Bohrung (16) angeordnet ist, und
- die beiden benachbarten Öffnungen (18, 22) im Bereich des vorderen Endes (12) angeordnet sind.

2. Knochenanker nach Anspruch 1,
**dadurch gekennzeichnet, dass**
die beiden benachbarten Öffnungen (18, 22) im Bereich einer Kante (13) des Körpers (10) angeordnet sind.

3. Knochenanker nach Anspruch 1,
**dadurch gekennzeichnet, dass**
eine Bohrung (14) quer zur Längsachse des Körpers (10) verläuft.

4. Knochenanker nach Anspruch 1,
**dadurch gekennzeichnet, dass**
beide Bohrungen (14, 16) unter einem Winkel von etwa 45° zueinander verlaufen.

5. Knochenanker nach Anspruch 1,
**dadurch gekennzeichnet, dass**
am Außenumfang des Körpers (10) zwischen zwei Öffnungen zweier Bohrungen eine Rille (26, 28) vorgesehen ist.

6. Knochenanker nach Anspruch 1,
**dadurch gekennzeichnet, dass**
am Außenumfang des Körpers (10) eine sich parallel zur Mittelachse des Körpers (10) erstreckende Rille (26) vorgesehen ist, die sich zwischen zwei Öffnungen (18, 22) und bis zu einer Stirnseite des Körpers (10) erstreckt.

7. Knochenanker nach Anspruch 1,
**dadurch gekennzeichnet, dass**
am Außenumfang des Körpers (10) eine sich parallel zur Mittelachse des Körpers erstreckende Rille (26) vorgesehen ist, deren Breite der Breite einer in die Rille mündenden Öffnung (18, 22) entspricht.

8. Knochenanker nach Anspruch 1,
**dadurch gekennzeichnet, dass**
am Außenumfang des Körpers (10) eine sich parallel zur Mittelachse des Körpers erstreckende Rille (28) vorgesehen ist, deren Breite kleiner ist als die Breite einer in die Rille (28) mündenden Öffnung (20, 24).

9. Knochenanker nach Anspruch 1,
**dadurch gekennzeichnet, dass**
am Außenumfang des Körpers (20) umlaufende Rillen vorgesehen sind, die konzentrisch zu dessen Längsachse verlaufen.

10. Knochenanker nach Anspruch 1,
**dadurch gekennzeichnet, dass**
durch die Bohrungen (14, 16) ein Faden (F) derart hindurchgeführt ist, dass jeweils ein freies Fadenende (F-1, F2) aus-den beiden benachbarten Öffnungen (18, 22) am Außenumfang des Körpers (10) austritt.

## Claims

1. A bone anchor for the refixing of soft tissue (M') to bone (K, S) with the help of a suture (F), consisting of an elongate and substantially cylindrical body (10) made in one piece from human or animal cortical bone whose front end (12) tapers conically and which has at least two passage bores (14, 16) each having two openings (18, 20; 22, 24), wherein
- both bores (14, 16) lie in one plane and extend toward one another at an angle;
- an opening (18) of one bore (14) is arranged adjacent to an opening (22) of the other bore (16);
the other opening (20) of the one bore (14) is arranged spaced apart from the other opening (24) of the other bore (16); and
- the two adjacent openings (18, 22) are arranged in the region of the front end (12).

2. A bone anchor in accordance with claim 1, **characterized in that** the two adjacent openings (18, 22) are arranged in the region of an edge (13) of the body (10).

3. A bone anchor in accordance with claim 1, **characterized in that** one bore (14) extends transversely to the longitudinal axis of the body (10).

4. A bone anchor in accordance with claim 1, **characterized in that** both bores (14, 16) extend at an angle of approximately 45°, to one another.

5. A bone anchor in accordance with claim 1, **characterized in that** a groove (26, 28) is provided at the outer periphery of the body (10) between two openings of two bores.

6. A bone anchor in accordance with claim 1, **characterized in that** a groove (26) is provided at the outer periphery of the body (10) which extends parallel to the central axis of the body (10) and extends between two openings (18, 22) and up to an end face of the body (10).

7. A bone anchor in accordance with claim 1, **characterized in that** a groove (26) is provided at the outer periphery of the body (10) which extends parallel to the central axis of the body and whose width corresponds to the width of an opening (18, 22) opening into the groove.

8. A bone anchor in accordance with claim 1, **characterized in that** a groove (28) is provided at the outer periphery of the body (10) which extends parallel to the central axis of the body and whose width is smaller than the width of an opening (20, 24) opening into the groove (28).

9. A bone anchor in accordance with claim 1, **characterized in that** peripheral grooves are provided at the outer periphery of the body (20) which extend concentrically to its longitudinal axis.

10. A bone anchor in accordance with claim 16, **characterized in that** a suture (F) is guided through the bores (14, 16) such that a respective free suture end (F1, F1) exits the two adjacent openings (18, 22) at the outer periphery of the body (10).

## Revendications

1. Ancrage osseux pour la refixation de tissus mous (M') sur des os (K, S) à l'aide d'un fil (F), constitué d'un corps d'un seul tenant allongé et essentiellement cylindrique (10) d'un os cortical humain ou animal, dont l'extrémité antérieure (12) se rétrécit de manière conique, et qui comporte au moins deux perçages traversants (14, 16) avec deux ouvertures respectives (18, 20 ; 22, 24), dans lequel
- les deux perçages (14, 16) sont disposés dans un plan et s'étendent sous un angle l'un par rapport à l'autre,
- une ouverture (18) d'un perçage (14) est agencée voisine d'une ouverture (22) de l'autre perçage (16),
- l'autre ouverture (20) du premier perçage (14) est agencée à distance de l'autre ouverture (24) de l'autre perçage (16), et
- les deux ouvertures voisines (18, 22) sont agencées dans la zone de l'extrémité antérieure (12).

2. Ancrage osseux selon la revendication 1, **caractérisé en ce que** les deux ouvertures voisines (18, 22) sont agencées dans la zone d'une arête (13) du corps (10).

3. Ancrage osseux selon la revendication 1, **caractérisé en ce qu'**un perçage (14) s'étend transversalement à l'axe longitudinal du corps (10).

4. Ancrage osseux selon la revendication 1, **caractérisé en ce que** les deux perçages (14, 16) s'étendent sous un angle d'environ 45° l'un par rapport à l'autre.

5. Ancrage osseux selon la revendication 1, **caractérisé en ce qu'**il est prévu une rainure (26, 28) à la périphérie extérieure du corps (10) entre deux ouvertures de deux perçages.

6. Ancrage osseux selon la revendication 1, **caractérisé en ce qu'**il est prévu une rainure (26) à la périphérie extérieure du corps (10), laquelle s'étend parallèlement à l'axe médian du corps (10) et s'étend entre deux ouvertures (18, 22) et jusqu'à une face frontale du corps (10).

7. Ancrage osseux selon la revendication 1, **caractérisé en ce qu'**il est prévu une rainure (26) à la périphérie extérieure du corps (10), laquelle s'étend parallèlement à l'axe médian du corps, et dont la largeur correspond à la largeur d'une ouverture (18, 22) débouchant dans la rainure.

8. Ancrage osseux selon la revendication 1, **caractérisé en ce qu'**il est prévu une rainure (28) à la périphérie extérieure du corps (10), laquelle s'étend parallèlement à l'axe médian du corps et dont la largeur est inférieure à la largeur d'une ouverture (20, 24) débouchant dans la rainure (28).

9. Ancrage osseux selon la revendication 1, **caractérisé en ce qu'**il est prévu des rainures circonférentielles à la périphérie extérieure du corps (20), qui s'étendent concentriquement à son axe longitudinal.

10. Ancrage osseux selon la revendication 1, **caractérisé en ce qu'**un fil (F) est passé à travers les perçages (14, 16) de telle manière qu'une extrémité libre respective (F1, F2) du fil sort des deux ouvertures voisines (18, 22) à la périphérie extérieure du corps (10).
